Europäisches Patentamt

European Patent Office

Office européen des brevets

(11) Publication number: **0 258 862**
**A2**

# EUROPEAN PATENT APPLICATION

(21) Application number: 87112689.2

(51) Int. Cl.⁴ **G01N 21/75 , C12Q 1/00**

(22) Date of filing: 31.08.87

(30) Priority: 03.09.86 JP 206002/86

(43) Date of publication of application:
**09.03.88 Bulletin 88/10**

(84) Designated Contracting States:
**BE CH DE FR GB LI NL SE**

(71) Applicant: **KONISHIROKU PHOTO INDUSTRY CO. LTD.**
**No. 26-2, Nishishinjuku 1-chome Shinjuku-ku Tokyo 160(JP)**

(72) Inventor: **Hidaka, Seiji**
**Konishiroku Photo Industry Co. Ltd. 1,**
**Sakuramachi**
**Hino-shi Tokyo(JP)**
Inventor: **Ishihara, Takashi**
**Konishiroku Photo Industry Co. Ltd. 1,**
**Sakuramachi**
**Hino-shi Tokyo(JP)**
Inventor: **Kobayashi, Morio**
**Konishiroku Photo Industry Co. Ltd. 1,**
**Sakuramachi**
**Hino-shi Tokyo(JP)**
Inventor: **Haga, Isao**
**Konishiroku Photo Industry Co. Ltd. 1,**
**Sakuramachi**
**Hino-shi Tokyo(JP)**

(74) Representative: **Henkel, Feiler, Hänzel & Partner**
**Möhlstrasse 37**
**D-8000 München 80(DE)**

(54) **Method for measuring concentration or activity of specific component in liquid to be tested.**

(57) Disclosed is a method for measuring the concentration or the activity of a specific component, which comprises measuring the concentration or the activity of the specific component in a liquid to be tested containing the specific component and a co-existing substance which forms the same substance as the final product formed corresponding to the concentration or the activity of the specific component by measuring the time change of the optical density (D) by use of a dry system analytical element, characterized in that the optical densities of at least two points are measured during the reaction time while the time change rate (dD/dt) of the above optical density is greater than 0 and constant.

According to the present invention, a method for measuring the concentration or the activity of a specific component by use of a dry system analytical element, which is improved in accuracy of analysis by removing errors derived from co-existing substances, is enabled.

# Method for measuring concentration or activity of specific component in liquid to be tested

## BACKGROUND OF THE INVENTION

This invention relates to a method for analyzing a specific component in a fluid sample, more particularly to a method for measuring activity or concentration of a specific component in a biological fluid sample by measuring the time change of the optical density (D) by use of a dry system analytical element.

The initial rate method as a measuring means for analyzing a specific component in a biological fluid sample is the method which performs quantitative determination by measuring the reaction rate and may be used for activity assay of an enzyme, and also in the case of coloration reaction in which the reaction is directly proportional to the concentration of a specific component. However, in some cases, due to interfering actions of co-existing substances in the above biological fluid sample, accurate analysis cannot be done. In the case of wet chemistry of the prior art, interfering actions of co-existing substances can be removed by reduction in the ratio of the liquid amount of the specific component relative to the total liquid amount to be reacted, addition of agents for inhibiting or removing co-existing substances, the order and the timing in which the reagents are to be added, etc. On the other hand, in the case of dry chemistry, since the reaction solution is generally only the water in the above biological fluid sample, it is susceptible to influences from co-existing substances. Also, although it is possible to incorporate agents for inhibiting or removing co-existing substances, all the reagents are in dry state and all the reactions proceed for the first time in the matrix containing reagents by encountering a fluid sample during measurement, and therefore the order and timing in which the reagents are to be added cannot be set and the interfering action of co-existing substances cannot necessarily be removed effectively. And, when the co-existing substances are the same as the intermediate products which are formed depending on the activity or the concentration of the above specific component, they can neither be inhibited nor removed. For example, the method using formazan color forming system has been well known in the art, and the co-existing substance such as glucose in measurement of $\alpha$-amylase, L-glutamic acid in measurement of GOT (glutamate oxaloacetate transaminase), GPT (glutamate pyruvate transaminase), glycerine in measurement of triglyceride, etc. is the same as the intermediate product depending on the activity or the concentration of each specific component.

In the case of wet chemistry, as disclosed in Japanese Unexamined Patent Publication No. 58097/1985, the above co-existing substance can be removed and thereafter the specific component can be analyzed with addition of a tetrazolium compound.

However, in the case of dry chemistry as described in detail in Japanese Unexamined Patent Publications Nos. 88097/1984 and 91896/1984, and Japanese Unexamined Patent Publication No. 262660/1986, the above co-existing substance cannot be removed, whereby errors are caused to occur.

Such co-existing substance may include pyruvic acid in GOT, GPT measurement by use of pyruvate oxidase as disclosed in Japanese Unexamined Patent Publication No. 47696/1985.

## SUMMARY OF THE INVENTION

An object of the present invention is to provide a method for measuring the concentration or the activity of a specific component by use of a dry system analytical element, which is improved in accuracy of analysis by removing errors derived from co-existing substances.

To outline the present invention, the present invention is an invention concerning the method for measuring the concentration or the activity of a specific component in a liquid to be tested, and it is a method for measuring the concentration or the activity of a specific component, which comprises measuring the concentration or the activity of said specific component in a liquid to be tested containing the specific component and a co-existing substance which forms the same substances as the final product formed corresponding to the concentration or the activity of said specific component by measuring the time change of the optical density (D) by use of a dry system analytical element, characterized in that the optical densities of at least two points are measured during the reaction time while the time change rate (dD/dt) of the above optical density is greater than 0 and constant.

## BRIEF DESCRIPTION OF THE DRAWINGS

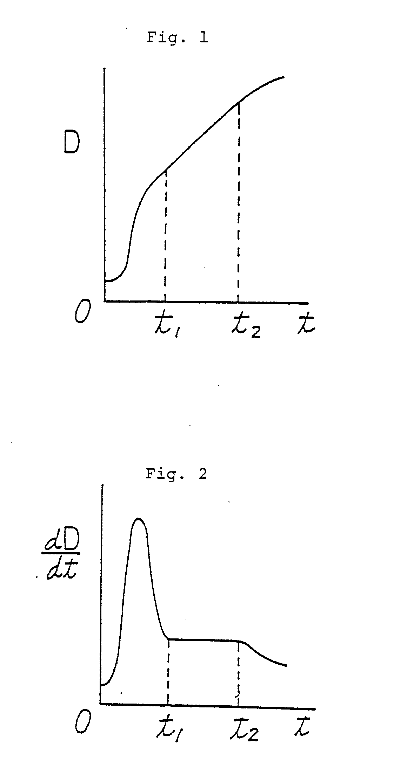

Fig. 1 is a graph showing an example of the relationship of the change in optical density with time of the dry system analytical element according to the present invention; and Fig. 2 is a graph showing the relationship of the change in the time change rate (dD/dt) of the optical density with time in that case.

## DETAILED DESCRIPTION OF THE INVENTION

The dry system analytical element according to the present invention is not limited in kind, provided that the reagents are contained in dry state and all the chemical reactions proceed in the matrix containing the reagents on encountering a fluid sample. For example, there is an element of a filter paper impregnated with reagents as disclosed in Japanese Patent Publication No. 39558/1975 and Japanese Unexamined Patent Publication No. 151096/1979. Also, there may be included an analytical element having successively at least one reagent layer and a porous spreading layer on a light-transmissive and liquid-impermeable support such as an analytical element having a spreading layer of a non-fibrous porous medium as disclosed in Japanese Patent Publication No. 21677/1978, an analytical element having a spreading layer of a fabric subjected to hydrophilic treatment as disclosed in Japanese Unexamined Patent Publication No. 164356/1980, analytical elements having spreading layers of fibrous structures as disclosed in Japanese Unexamined Patent Publications Nos. 94658/1982 and 70161/1983 and U.S Patents Nos. 4,594,224 and 4,427,632, and an analytical element having a spreading layer of a bound particulate structure as disclosed in Japanese Unexamined Patent Publication No. 90167/1983.

The optical density according to the present invention is reflective optical density, which is measured by a reflection type spectrophotometer by use of an integral sphere or optical fiber method, spectrophotometer, etc.

In the present invention, all the specific components which can be analyzed by the rate assay method which performs quantitation by measurement of the reaction rate. For example, there may be included GOT, GPT, LDH (lactate dehydrogenase), ALP (alkaline phosphatase), LAP (leucine aminopeptidase), $\gamma$-GTP ($\gamma$-glutamyl transpeptidase), CPK (creatine phosphokinase), etc.

The final product formed on the above specific component according to the present invention differs depending on the reaction system employed. Accordingly, the co-existing substance which gives the same final product as said final product also differs depending on the reaction system employed. For example, as described in Japanese Unexamined Patent Publications Nos. 88097/1984 and 91896/1984, and Japanese Unexamined Patent Publication No. 104776/1985 as mentioned above, when using a tetrazolium compound as the dye forming precursor, the final product is a formazan dye, and the co-existing substance may be ascorbic acid and lactic acid, and also glucose in $\alpha$-amylase measurement, further L-glutamic acid in GOT and GPT measurements, and further glycerine in triglyceride measurement, etc. Also, in the case of the element for measurement of GOT and GPT which forms hydrogen peroxide as described in the above Japanese Unexamined Patent Publication No. 47696/1985, the final product is a quinone type dye, and the co-existing substance in the liquid to be tested which gives the same final product may include oxaloacetic acid and pyruvic acid. Otherwise, when using a reduction type co-enzyme as disclosed in Japanese Unexamined Patent Publication No. 56099/1986, the final product is nicotinamidoadenine dinucleotide (NAD$^+$) which is an oxidation type co-enzyme, and the co-existing substance in the liquid to be tested which gives this NAD$^+$ may include pyruvic acid which is a substrate for LDH. Further, in the measurement of creatinine by enzyme method, the co-existing substance is creatine.

An example of the time change of the optical density (D) according to the present invention is shown as a graph in relationship between D (axis of ordinate) and time (t, axis of abscissa) in Fig. 1. Also, in Fig. 2, the time change in the time change rate (dD/dt) of the optical density corresponding to Fig. 1 is shown as a graph in relationship between dD/dt (axis of ordinate) and time (t, axis of abscissa). The interval of the reaction time when dD/dt in the present invention is greater than 0 and constant indicates the time from $t_1$ to $t_2$ when there is no influence of the interfering action of co-existing substances. Although its time depends on the reaction conditions, when measurement is conducted by incubation at 37 °C of the analytical element having a spreading layer of fibrous structure as disclosed in Japanese Unexamined Patent Publications Nos. 94658/1982 and 70161/1983 and U.S. Patents Nos. 4,594,224 and 4,427,632, it is desirable to perform measurements at at least two points from 4 minutes to 20 minutes later, preferably from 6 minutes to 15 minutes later. In the case of the two point measurement, the time interval may be desirably 1 minute or longer, preferably 2 minutes or longer. By setting such an automatic analytical instrument, accurate analysis can be done without errors at the initial stage.

The present inventon is described in more detail by referring to Examples, by which the present invention is not limited at all.

Example - 1 (Analytical element for GOT)

On a transparent polyethylene terephthalate support subjected to subbing treatment with a film thickness of 180 μm was provided a first reagent layer having the following composition.

First reagent layer:

Gelatin    21.0 g/cm²
Glutamic acid dehydrogenase    42,000 U/m²
Diaphorase    2,100 U/m²
3,3'-(4,4'-biphenylene)-bis(2,5-diphenyltetrazoliumchloride)    1.0 g/m²
Triton X-100 (Rohm & Haas Co.)    2.1 g/m²
1,2-bis(vinylsulfonyl)ethane    0.15 g/m²

On the above first reagent layer, further the following second reagent layer and the spreading layer were provided successively to prepare the analytical element of the present invention.

Second reagent layer:

Tris-hydroxymethylaminomethane    5.70 g/m²
HCl-Tris-hydroxymethylaminomethane    1.22 g/m²
Luviscol VA-28*1)    2.0 g/m²
Triton X-100 *    0.5 g/m²
*1) trade name of BASF Co.: N-vinylpyrrolidone-vinyl acetate copolymer (molar ratio = 20: 80).
* the above second reagent layer was coated by sand grinder direct dispersion by use of n-butanol as the solvent.

Spreading layer:

Powdery filter paper (Toyoroshi K.K., 40 to 100 mesh)    91 g/m²
Monosodium aspartate    4.1 g/m²
α-Ketoglutaric acid    0.5 g/m²
Oxidized type nicotinamidoadeninedinucleotide (NAD⁺)    5.5 g/m²
Styrene-glycidyl methacrylate copolymer (weight ratio = 9:1)    22 g/m²
Triton X-100    9 g/m²
*the above spreading layer components was coated with xylene solvent
*Monosodium aspartate and NaD⁺ were coated separately by direct dispersion with sand grinder by use of xylene as the solvent
*α-ketoglutaric acid was dissolved in methanol before addition.

After 200 test samples of human serum were added dropwise each in 10 μl on the above analytical element on the spreading layer, incubation was effected at 37 °C and the reflective densities at the two points after elapse of the respective times shown in Table - 1 were measured through a filter of 546 nm by a reflective spectrophotometer to determine the difference in the reflective densities, and the correlation between the respective values of 200 test samples and the Hitachi Automatic Analyzer 706D was determined to obtain the results shown in Table - 2.

Table - 1

|  | First measurement | Second measurement |
|---|---|---|
| Measuring method of |  |  |
| Invention - 1 | 4 min. | 6 min. |
| " - 2 | 5 min. | 7 min. |
| " - 3 | 7 min. | 11 min. |
| Measuring method of |  |  |
| Control - 1 | 2 min. | 4 min. |

Table - 2

|  | Correlation coefficient |
|---|---|
| Measuring method of |  |
| Invention - 1 | 0.952 |
| " - 2 | 0.973 |
| " - 3 | 0.996 |
| Measuring method of |  |
| Control - 1 | 0.752 |

As is apparent from the results in Table - 2, it can be understood that as contrasted to the poor correlation of the measuring method - 1 of Control due to interference by co-existing substance, the measuring methods - 1 to 3 of the present invention with the first measurement of 4 minutes and thereafter are substantially uninfluenced therefrom, thus being improved in accuracy of analysis.

Example - 2 (Analytical element for GPT)

On a transparent polyethylene terephthalate support subjected to subbing treatment with a film thickness of 180 μm, a first reagent layer with the following composition was provided.

First reagent layer

Gelatin    21.0 g/m$^2$
Glutamine dehydrogenase    21,000 U/m$^2$
Diaphorase    2,100 U/m$^2$
3,3'-(4,4'-Biphenylene)-bis(2,5-diphenyltetrazoliumchloride)    1.0 g/m$^2$
Triton X-100    2.1 g/m$^2$
1,2-Bis(vinylsulfonyl)ethane    0.15 g/m$^2$

On the above first reagent layer, the following second reagent layer and spreading layer were further provided successively to prepare the analytical element of the present invention.

5

Second reagent layer:

Tris-hydroxymethylaminomethane     5.70 g/m²
HCl-Tris-hydroxymethylaminomethane     1.22 g/m²
Luviscol VA-28     3.0 g/m²
Triton X-100     0.5 g/m²
*the above second reagent layer was coated by sand grinder direct dispersion by use of n-butanol as the solvent.

Spreading layer (S):

Powdery filter paper [Toyo Roshi K.K.
40 to 100 mesh]     91 g/m²
Alanine     2.2 g/m²
$\alpha$-Ketoglutaric acid     0.25 g/m² Oxidized type nicotinamide adenine
dinucleotide (NAD⁺)     5.5 g/m²
Styrene-glycidyl methacrylate
copolymer (weight ratio = 9:1)     22 g/m²
Triton X-100     9 g/m²
*the above spreading layer components was coated with xylene solvent
*alanine and NAD⁺ were added separately by direct dispersion with sand grinder with the use of xylene as the solvent
*$\alpha$-ketoglutaric acid was dissolved in methanolxylene solvent, then powdery paper was dipped and impregnated with the solution under stirring, filtered and dried before use.

After each 10 $\mu$l of the dialyzed pooled sera added with GPT (Sigma Co., derived from Porcine Heart) to which was further added 0, 10, 20 mg/dl of ascorbic acid and each of the pooled sera to which was added 0, 5, 10 mg/dl of L-glutamic acid was added dropwise on the above analytical element on the spreading layer, incubation was effected at 37 °C and the reflective optical densities at the two points after elapse of time indicated in Table - 1 were measured through a filter of 546 nm by a reflective spectrophotometer to obtain the results shown in Table - 3 and Table - 4.

Table - 3

| Amount of ascorbic acid | 0 (mg/dl) | 0 (mg/dl) | 0 (mg/dl) |
|---|---|---|---|
| Measuring method of Invention - 1 | 0.035 | 0.036 | 0.038 |
| " - 2 | 0.033 | 0.033 | 0.034 |
| " - 3 | 0.040 | 0.040 | 0.041 |
| Measuring method of Control - 1 | 0.030 | 0.039 | 0.051 |

Table - 4

| Amount of L-glutamic acid | 0 (mg/dl) | 0 (mg/dl) | 0 (mg/dl) |
|---|---|---|---|
| Measuring method of Invention - 1 | 0.035 | 0.040 | 0.047 |
| " - 2 | 0.033 | 0.036 | 0.040 |
| " - 3 | 0.040 | 0.040 | 0.041 |
| Measuring method of Control - 1 | 0.030 | 0.043 | 0.061 |

As is apparent from the results in Table - 4, it can be understood that, while the measurement method of Control - 1 receives great influence from L-glutamic acid, the measuring methods of the present invention 1 to 3 receive substantially no influence therefrom, thus being improved in accuracy of analysis. Also, from the results in Table - 3, it can be understood that, while the method of Control - 1 receives great influence from ascorbic acid, the measureing methods of the present invention 1 - 3 receive substantially no influence therefrom, thus being improved in accuracy of analysis without addition of a removing agent such as ascorbic acid oxidase.

As described above, the method of the present invention is extremely useful in removing the influence of co-existing substances by a simple method to enable accurate analysis without superfluous use of reagents.

**Claims**

1. A method for measuring the concentration or the activity of a specific component, which comprises measuring the concentration or the activity of said specific component in a liquid to be tested containing the specific component and a co-existing substance which forms the same substance as the final product formed corresponding to the concentration or the activity of said specific component by measuring the time change of the optical density (D) by use of a dry system analytical element, characterized in that the optical densities of at least two points are measured during the reaction time while the time change rate (dD/dt) of the above optical density is greater than 0 and constant.

2. The method for measuring the concentration or the activity of a specific component according to Claim 1, wherein the specific component is glutamate oxaloacetate transaminase, glutamate pyruvate transaminase, lactate dehydrogenase, alkaline phosphatase, leucine aminopeptidase, γ-glutamyl transpeptidase or creatine phosphokinase.

3. The method for measuring the concentration or the activity of a specific component according to Claim 1, wherein the optical density is measured by a reflection type spectrophotometer.

7

4. The method for measuring the concentration or the activity of a specific component according to Claim 1, wherein the time between the at least two points during the reaction time is not less than 1 minute.

5. The method for measuring the concentration or the activity of a specific component according to Claim 4, wherein the time between the at least two points during the reaction time is not less than 2 minutes.

Fig. 1

Fig. 2